Europäisches Patentamt

European Patent Office

Office européen des brevets

(11) Publication number: **0 490 147 A2**

## EUROPEAN PATENT APPLICATION

(21) Application number: **91120011.1**

(22) Date of filing: **23.11.91**

(51) Int. Cl.⁵: **C07C 19/08**, C07C 17/00

(30) Priority: **12.12.90 US 626345**

(43) Date of publication of application:
**17.06.92 Bulletin 92/25**

(84) Designated Contracting States:
**CH DE FR GB IT LI NL**

(71) Applicant: **HALOCARBON PRODUCTS CORPORATION**
**120 Dittman Court, N. Augusta Industrial Park, P.O. Box 6369**
**N. Augusta, S.C.(US)**

(72) Inventor: **Sprague, Lee G.**
**909 Holiday Drive**
**North Augusta, SC 29841(US)**

(74) Representative: **Klöpsch, Gerald, Dr.-Ing.**
**Patentanwälte Klöpsch & Flaccus et al**
**An Gross St. Martin 6**
**W-5000 Köln 1(DE)**

(54) Preparing isomer free 1,1-dichloro-2,2,3,3,3-pentafluoropropane.

(57) In the reduction of a trichloropentafluoropropane to produce 1,1-dichloro-2,2,3,3,3-pentafluoropropane, the improvement which comprises effecting the reduction with a sulfite in an aqueous medium.

EP 0 490 147 A2

## BACKGROUND OF THE INVENTION

1,1,2-Trichloro-1,2,2-trifluoroethane (R-113) has found extensive use as a cleaning solvent for the removal of flux from printed wiring boards and as a degreaser of precision and polished surfaces. However, R-113 is a fully halogenated chlorofluorocarbon and has been implicated in the destruction of the ozone layer. Consequently, its production is being phased-out. Alternative, ozone-friendly solvents are needed as substitutes. Such substitutes must also be non-toxic and inexpensive.

One proposed substitute for R-113 is 1,1-dichloro-2,2,3,3,3-pentafluoropropane (R-225ca, $CF_3CF_2CHCl_2$). Unfortunately, the most direct method of making R-225ca also results in the production of the isomeric R-225cb ($CF_2ClCF_2CHClF$). For example, the reaction of tetrafluoroethylene and dichlorofluoromethane in the presence of aluminum chloride has been disclosed (Coll. Czech. Chem. Commun., Vol. 36, p 1867, 1971) to give R-225ca (59 parts) and R-225cb (41 parts). The isomers have close boiling points and could not be separated by distillation. The R-225cb is thermodynamically less stable and this may cause problems during the use of the isomeric mixture. Other methods of making R-225ca are unattractive commercially. For example, US 3,381,042 discloses the production of R-225ca by the reaction of fluoride ion, tetrafluoroethylene and chloroform. While the product is free of isomers, the reaction has to be conducted in a solvent such as diethyleneglycol dimethyl ether, a limitation which is not industrially volume efficient. Other methods described in the literature, such as J. Amer. Chem. Soc., Vol. 77, p 3149 (1955) and J. Chem. Soc., p 2193 (1957) involve multistep process which are not commercially feasible. There is a need, therefore, for improved processes wherein R-225ca is produced cheaply and free of isomer contamination.

The above mentioned Czechoslovakian reference also discloses that the addition of fluorotrichloromethane to tetrafluoroethylene in the presence of aluminum chloride produces a mixture of trichloropentafluoropropanes as $CF_3CF_2CCl_3$ (83 parts) and $CF_2ClCF_2CFCl_2$ (17 parts). Upon reduction with isopropanol in the presence of actinic light, a mixture of the R-225ca and R-225cb was obtained. Surprisingly it has now been found that if the reduction of these isomeric trichloropentafluoropropanes is done with aqueous sulfite ion, only the $CF_3CF_2CCl_3$ reacts and there is obtained, after distillation, R-225ca free of isomer contamination.

The sulfite ion reduction of halogenated compounds is known. East German patent 40,475 discloses the debromination of $CF_3CClBr_2$ with so-

dium sulfite, sodium bisulfite or sodium dithionate in aqueous solution to give $CF_3CHClBr$. German patent DE 3,834,038 discloses the sulfite reduction of $CF_3CCl_3$ to give $CF_3CHCl_2$. No mention is made of the lack of reactivity of isomers under these conditions. There is no prior art to indicate that sulfite reductions are isomer specific.

## DETAILED DESCRIPTION OF THE INVENTION

The process of the present invention involves bringing into contact the $CF_3CF_2CCl_3$, either substantially pure or optionally mixed with $CF_2ClCF_2CFCl_2$, with an aqueous solution of sodium sulfite or bisulfite. The process is accompanied by the production of hydrogen chloride which is optionally neutralized by reaction with a base. Suitable bases include, but are not limited to, alkali metal hydroxides, carbonates, bicarbonates and oxides, primary, secondary and tertiary amines, and the like. The reduction agent is optionally any water soluble sulfite or bisulfite. The reducing reagent may be made by dissolution of the salt in water or preferably by the addition of sulfur dioxide to a solution of sodium or potassium hydroxide in water. The amount of reducing agent employed in the process should be at least equimolar to the amount of $CF_3CF_2CCl_3$, preferably one to too moles per mole of $CF_3CF_2CCl_3$. Optionally a phase transfer catalyst such as Aliquat$^R$ may be employed.

The reaction is usually conducted at temperatures in the range of 50°C to 150°C, preferably 80°C to 120°C, optionally at superatmospheric pressure. Optionally the reactor is fitted with a distillation column to remove the lower boiling product as it is formed from the starting material.

The invention will be further described in the following illustrative examples wherein all parts are by weight unless otherwise expressed.

### EXAMPLE 1

Aluminum chloride (10.5 g) was added to a 500 ml stirred autoclave which was then sealed, evacuated and charged with trichlorofluoromethane (350 g). To this stirred mixture, initially at 20°C, was added tetrafluoroethylene (184 g) at such a rate as to maintain the pressure below 100 psig and temperature at less than 30°C, with cooling provided by internal water cooled coils as well as an external ice-bath. After approximately two hours the autoclave was cooled to 5°C, opened and the liquid was decanted from the aluminum halides. The liquid was washed with water and distilled to give 302 g of organic product comprised of 80.1% $C_3Cl_3F_5$, 16.8% $CCl_4$ and 1.8% $CFCl_3$ by glc. The NMR revealed that the $C_3Cl_3F_5$ consisted of 85%

$CF_3CF_2CCl_3$ and 15% $CF_2ClCF_2CFCl_2$.

**EXAMPLE 2**

In a typical procedure, 44.1 g of the product from Example 1 was added to a stirred autoclave containing 18.5% sodium sulfite solution (305 ml) and the mixture was heated to 100°C for six hours. The cooled autoclave was opened and the contents transferred to a separatory funnel. The organic layer was separated and distilled to give $CF_3CF_2CHCl_2$, free of $CF_2ClCF_2CHClF$ by NMR: $^1$H NMR 5.91 ppm (t, $-CF_2CHCl_2$) $^3J_{H,F}$ = 9.0 Hz; $^{19}$F NMR -80.07 ppm (s, 3F, $-\overline{C}F_3$), -120.73 ppm (d, 2F, $-CF_2-$).

It will be appreciated that the instant specification and claims are set forth by way of illustration and not limitation, and that various modifications and changes may be made without departing from the spirit and scope of the present invention.

**Claims**

1. In the reduction of a trichloropentafluoropropane to produce 1,1-dichloro-2,2,3,3,3-pentafluoropropane, the improvement which comprises effecting the reduction with a sulfite in an aqueous medium.

2. The process according to claim 1, wherein the trichloropentafluoropropane comprises $CF_3CF_2CCl_3$.

3. The process according to claim 1, wherein the trichloropentafluoropropane comprises a mixture of isomers including $CF_3CF_2CCl_3$.

4. The process according to claim 1, wherein the reduction is effected with an aqueous solution of sodium sulfite or bisulfite.